# FASCICULE DE BREVET EUROPEEN

(11) **EP 2 276 521 B1**
(45) Date de publication et mention de la délivrance du brevet: **09.07.2014**
(21) Numéro de dépôt: 09757732.4
(22) Date de dépôt: 14.05.2009
(51) Int. Cl.: A61M 1/34, A61M 1/36, B01D 63/08

(54) **DISPOSITIF DE FILTRATION D'UN LIQUIDE COMPLEXE TEL QUE DU SANG, NOTAMMENT APPLICABLE A UN AUTOTRANSFUSEUR**
VORRICHTUNG ZUR FILTERUNG EINER KOMPLEXEN FLÜSSIGKEIT, Z B. BLUT, INSBESONDERE ZUR VERWENDUNG MIT EINEM AUTOTRANSFUSIONSGERÄT
DEVICE FOR FILTERING A COMPLEX LIQUID SUCH AS BLOOD, PARTICULARLY APPLICABLE TO AN AUTOTRANSFUSER

(30) Priorité: 14.05.2008 FR 0853112
(43) Date de publication de la demande: 26.01.2011
(73) Titulaire: Direction et Priorités SA, 33170 Gradignan (FR)
(72) Inventeur: GADRAT, Francis, F-33200 Bordeaux (FR); CHASTENET, Bertrand, F-33320 Eysines (FR)
(74) Mandataire: Fantin, Laurent
(86) Numéro de dépôt international: PCT/FR2009/050903
(87) Numéro de publication internationale: WO 2009/147356

(56) Documents cités:
- DE-U1- 9 104 912
- FR-A- 2 371 954
- FR-A- 2 554 010
- JP-A- 2 095 421
- US-A- 4 411 792
- US-A- 4 935 002

## Description

La présente invention concerne un dispositif de filtration d'un liquide complexe tel que du sang notamment apte à être intégré dans un dispositif de traitement du sang en vue d'une autotransfusion.

On sait que la filtration du sang afin de retirer les impuretés ou certains composés notamment dans le cas d'une autotransfusion pose de nombreux problèmes notamment de colmatage des surfaces filtrantes.

Les pores de ces surfaces filtrantes modifient au fur et à mesure les conditions de filtration si bien que le débit de filtration diminue jusqu'à devenir nul.

Pour la suite de la description, l'exemple retenu est le sang qui est un milieu hétérogène, le dispositif de filtration selon la présente invention trouvant application pour toute composition liquide ayant des problématiques identiques. C'est ainsi que dans le sang, on trouve la présence de protéines de masses moléculaires plus ou moins importantes mais aussi des éléments cellulaires. Si l'on prévoit un seuil de filtration pour les éliminer, ces chaînes protéiques et éléments cellulaires viennent obturer les pores de la surface de filtration interdisant le passage des éléments à filtrer voire même de l'eau.

On constate aussi que la perméation dans les membranes existantes se produit perpendiculairement à la surface, le volume liquide à filtrer étant disposé au-dessus de la membrane et le filtrat étant en dessous. Une mise sous vide permet aussi de favoriser le passage sauf s'il se produit un colmatage car alors la dépression favorise le plaquage de la couche de colmatage et accentue au contraire le phénomène.

Le phénomène peut aussi être aggravé par la sédimentation de certains éléments composant la phase liquide à filtrer. Dans ce cas, la gravité a tendance à faire accumuler ces éléments sur la surface filtrante, accélérant le phénomène de colmatage, d'autant plus s'il y a dépression. Une solution consisterait à agiter la composition à filtrer mais ceci n'est pas facile à réaliser si l'on recherche des systèmes simples.

Si l'on souhaite par exemple intégrer un tel dispositif de filtration par exemple dans un autotransfuseur tel que celui décrit dans la demande de brevet français N°-2.874.327, de tels moyens d'agitation ne sont pas envisageables.

Un filtre pour l'autotransfusion est connu du brevet US 4,935,002.

Il convient de proposer un agencement qui permet une filtration d'une quantité de composition liquide complexe avec un débit qui ne diminue que faiblement au cours du temps, qui reste très compact pour autoriser une intégration par exemple dans un dispositif autonome d'autotransfusion, qui soit d'un prix de revient faible pour pouvoir s'adresser au plus grand nombre, qui utilise les membranes de filtration existantes afin d'éviter le développement de membranes spécifiques et qui puisse être agréé par les autorités médicales.

C'est le but du dispositif de filtration selon la présente invention, qui est définie par la revendication 1.

Le dispositif de filtration est maintenant décrit en détail suivant un mode de réalisation particulier, non limitatif en regard des dessins annexés sur lesquels les différentes figures représentent :
- figure 1 : une vue en perspective schématique du dispositif selon l'invention,
- figure 2 : une vue en coupe transversale verticale suivant la ligne 2-2 de la figure 1
- figure 3 : une vue en coupe transversale verticale suivant la ligne 3-3 de la figure 1,
- figure 4 : une vue schématique d'une installation équipée du dispositif selon la présente invention,
- figure 5 : une vue en coupe d'un agencement notamment adapté pour être associé à un autotransfuseur.

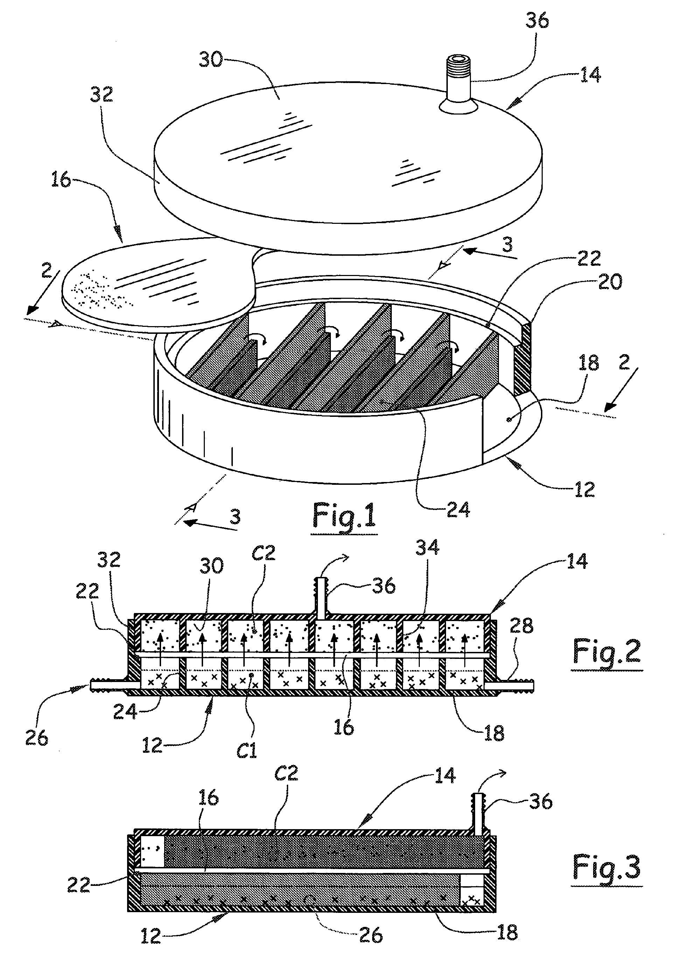

Sur la figure 1, on a représenté un dispositif de filtration d'un liquide complexe 10, ledit dispositif comprenant une embase 12 et une tête 14 apte à venir s'emboîter dans ladite embase.

Les différents éléments sont avantageusement réalisés en matière plastique apte à être moulée avec précision et présentant une compatibilité médicale et/ou alimentaire suivant les applications envisagées. Dans le cas présent pour une application à la filtration du sang, notamment en vue d'une autotransfusion, la matière plastique dispose de toutes les aptitudes à répondre aux normes concernées.

L'embase 12 et la tête 14 sont donc venues de moulage avec une précision telle que l'emboîtement à frottement doux assure une étanchéité lorsque les deux parties sont totalement emboîtées ainsi qu'il sera expliqué après.

Entre l'embase 12 et la tête 14, il est interposée une membrane 16 de filtration. Cette membrane 16 a un seuil de coupure adapté au produit à filtrer comme il sera expliqué plus avant dans la description.

L'embase 12 est de type cylindrique avec un fond 18 et une paroi 20 annulaire. Cette paroi 20 annulaire comporte un rebord 22 définissant un premier diamètre intérieur d et un second diamètre intérieur D. Le second diamètre D correspond au diamètre extérieur de la tête 14, au jeu d'emboîtement étanche près.

Le fond 18 comprend des nervures 24 venues de moulage avec ledit fond. Ces nervures ont une hauteur telle qu'elles viennent au droit du rebord 22. Ces nervures 24 forment des chicanes étant alternativement issues de la paroi 20 circulaire par une de leur extrémité sans atteindre cette même paroi 20 circulaire à l'autre extrémité.

L'embase 12 comporte un piquage 26 d'entrée, dont la direction d'introduction du fluide est orientée perpendiculairement au plan des nervures 24.

Le piquage 26 d'entrée est implanté en dessous du rebord 22 pour déboucher dans la chambre C1 basse, définie par la membrane 16, le fond 18 de l'embase 12 et la paroi 20 annulaire de cette même embase.

L'embase 12 comporte également un piquage 28 de sortie, disposé diamétralement opposé au piquage 26 d'entrée.

Le piquage 28 de sortie est implanté en dessous du rebord 22 pour sortir de la chambre C1 basse, définie par la membrane 16, le fond 18 de l'embase 12 et la paroi 20 annulaire de cette même embase.

La tête 14 comprend un fond 30 et une paroi 32 annulaire.

La paroi 32 annulaire a une épaisseur sensiblement égale à la largeur du rebord 22 de l'embase 12.

La membrane 16 est ainsi au moins immobilisée périphériquement entre le rebord 22 de l'embase 12 et la paroi 32 annulaire de la tête 14, comme cela est visible sur les figures 2 et 3.

La tête 14 est munie de nervures 34, venues de moulage avec le fond. Ces nervures sont également venues de moulage par une extrémité de la paroi 32 annulaire, sans atteindre par l'autre extrémité cette même paroi 32 annulaire. Ces nervures 34 forment donc un jeu de chicanes équivalent à celui des chicanes 24 de l'embase 12.

Les chicanes 24 de l'embase 12 et les nervures 34 de la tête sont en vis-à-vis de façon à immobiliser la membrane 16. L'homme de l'art ajustera les hauteurs des nervures pour assurer un serrage adapté après montage, ceci en fonction de l'épaisseur de ladite membrane notamment mais aussi de sa nature.

Un piquage 36 de sortie est ménagé dans le fond 30 de cette tête.

Une seconde chambre C2 haute est définie par la membrane 16, le fond 30 et la paroi 32 annulaire.

On constate que la membrane 16 est non seulement maintenue par sa périphérie mais aussi entre les nervures 24 de l'embase 12 et les nervures 34 de la tête 14.

Ainsi, la membrane est parfaitement maintenue.

De façon avantageuse, il est possible de prévoir sur le rebord 22 de l'embase 12 une rainure et sur la tranche de la paroi 32 annulaire de la tête une languette de profil conjugué de celui de la rainure de sorte à améliorer encore l'immobilisation. Ce perfectionnement n'est qu'un des moyens d'améliorer le serrage, tout autre moyen équivalent étant inclus dans la présente demande.

Le dispositif selon la présente invention est mis en oeuvre de la façon suivante. Suivant les applications, les modules sont réalisés avec la membrane 16 ayant le seuil de coupure recherché.

Chaque dispositif est avantageusement réalisé en usine, sous conditions adaptées pour le matériel médical lorsque c'est sa vocation.

Les deux parties, embase 12 et tête 14, sont emboîtées après avoir disposé la membrane 16.

Avantageusement, une soudure périphérique par ultrasons ou laser permet de souder les deux parties en matière plastique et assure une étanchéité totale. Cette soudure périphérique très précise et localisée ne provoque qu'un apport de chaleur négligeable et ne provoque aucune dégradation de la membrane elle-même.

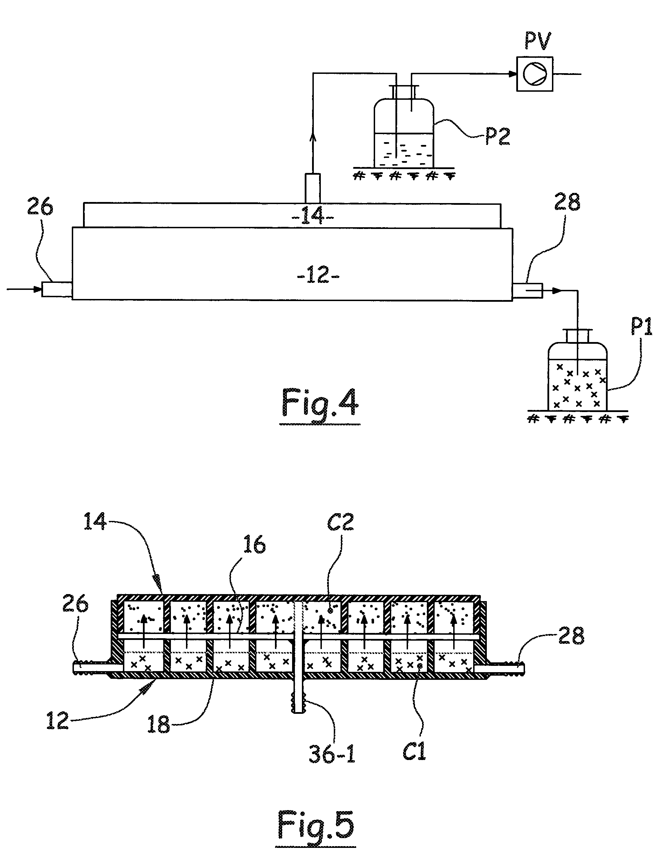

En se reportant à la figure 4 en complément des trois premières, l'opérateur connecte le piquage 26 sur l'alimentation en composition à purifier, par exemple une composition sanguine issue d'un drain suite à une intervention chirurgicale. Le piquage 28 de sortie de l'embase 12 est relié à un contenant P1 prévu pour recueillir le rétentat, c'est-à-dire les éléments retenus par le filtre.

Le piquage 36 de la tête est relié à un contenant P2 prévu pour recueillir le filtrat à éliminer. Ce contenant P2 est avantageusement mis sous dépression par un raccord sur une source de vide PV.

Le piquage 36 de sortie de la chambre C2 haute est donc soumis à dépression si nécessaire.

La composition est introduite dans la chambre C1 basse et circule dans le jeu de chicanes des nervures 24.

Le piquage 28 de sortie comprend des moyens de réglage du débit afin de créer une légère pression dans la chambre C1 du filtre si l'on réduit un peu le débit.

Il faut tenir compte de la perte du volume du perméat passé à travers la membrane qui diminue le débit à travers le piquage 28 et de la perte de pression transmembranaire.

Du fait de cette circulation ralentie, il se produit également une sédimentation des particules les plus lourdes. De plus la circulation à travers les chicanes provoque une ségrégation qui tient les particules les plus lourdes au coeur du flux ou en partie basse tandis que le fluide peu chargé reste en surface. Parallèlement, la composition circule contre la membrane et sous l'effet des différents paramètres, équilibrage des débits et de la légère dépression, l'essentiel des particules de petites dimensions et les fluides passent tangentiellement à la membrane 16 et la traversent pour arriver dans la chambre C2 tandis que les particules de taille supérieure au seuil de coupure restent dans la chambre C1 et ce fluide chargé sort par le piquage 28 de sortie de l'embase 12.

Cette perméation se prolonge tout au long de la circulation de la composition dans la chambre C1 si bien qu'une particule qui n'est pas immédiatement aspirée à travers la membrane 16 alors qu'elle pourrait l'être, le sera potentiellement tout au long de la pérégrination de ladite composition le long de la membrane 16. On constate que les particules sédimentées et de dimensions adaptées passent à travers la membrane et ne peuvent pas colmater ladite membrane, ce qui est particulièrement avantageux.

Quant aux autres particules, de taille trop importante pour traverser la membrane, elles ne s'accumulent pas contre la paroi de la membrane du fait de la circulation tangentielle et de la gravité, limitant donc très fortement le colmatage et la diminution de débit de perméation.

Il est important de ne pas générer une dépression trop importante et un débit trop rapide car les particules seraient plaquées contre la membrane comme dans les agencements de l'art antérieur.

Toute la surface de la membrane est sollicitée, améliorant fortement le rendement pour une surface donnée de filtration.

De ce fait, ce type de dispositif de filtration peut être d'un encombrement réduit pour une capacité de filtration équivalente à celle d'un filtre de l'art antérieur.

Sur la figure 5, on a représenté un mode de réalisation avec un piquage 36-1, inversé, les autres éléments étant identiques.

Ce piquage 36-1 traverse de façon étanche le fond 18 de l'embase 12 et comprend des perforations dans sa partie située au sein de la chambre haute C2. La membrane 16 est également perforée pour laisser passer ce piquage 36-1, la membrane étant ajustée de façon étanche autour dudit piquage 36-1.

Un tel agencement permet une intégration aisée dans un dispositif d'autotransfusion tel que celui de la demande de brevet français N° 2 874 327. De la même façon la fraction concentrée ou rétentat sera retirée à la sortie par le piquage 28 de sortie tandis que le perméat sera retiré par le piquage 36-1.

Un tel dispositif de filtration de composition complexe est très avantageux en terme de fabrication également ne faisant appel qu'à des techniques connues et la membrane filtrante n'a pas besoin d'être particulière si ce n'est en dimensions, ce qui n'est pas un problème. Les membranes existantes disposent d'une résistance mécanique tout à fait suffisante compte tenu du fait qu'elle est parfaitement supportée par les nervures et par la périphérie.

On note aussi que du fait du non colmatage du filtre le débit varie très peu et le temps de traitement de la composition complexe est rapide.

## Revendications

1. Dispositif de filtration d'une composition fluide complexe tel que du sang, **caractérisé en ce qu'**il comprend une embase (12) avec un fond (18), une tête (14) avec un fond (30) et une membrane (16) disposée entre ladite embase et ladite tête de façon à définir une chambre C1 basse et une chambre C2 haute, un piquage (26) d'entrée de la composition complexe débouchant dans la chambre C1 basse, un piquage (28) de sortie débouchant de cette même chambre C1 basse et un piquage (36, 36-1) de sortie de la chambre C2 haute, le piquage (36,36-1) de sortie de la chambre C2 haute étant relié à une source de vide PV.

2. Dispositif de filtration d'une composition fluide complexe selon la revendication 1, **caractérisé en ce que** l'embase (12) comprend des nervures (24) formant un jeu de chicanes.

3. Dispositif de filtration d'une composition fluide complexe selon la revendication 2, **caractérisé en ce que** les nervures (24) sont orientées perpendiculairement au piquage (26) d'entrée.

4. Dispositif de filtration d'une composition fluide complexe selon la revendication 1, 2 ou 3, **caractérisé en ce que** la tête (14) comprend des nervures (34) formant un jeu de chicanes.

5. Dispositif de filtration d'une composition fluide complexe selon les revendications 3 et 4, **caractérisé en ce que** les nervures (24) de l'embase (12) et les nervures (34) de la tête (14) sont en vis-à-vis de façon à immobiliser la membrane (16).

6. Dispositif de filtration d'une composition fluide complexe selon l'une quelconque des revendications précédentes, **caractérisé en ce que** l'embase (12) comprend un rebord (22) destiné à recevoir la paroi (32) annulaire de la tête (14) de sorte à assurer l'immobilisation périphérique de la membrane (16).

7. Dispositif de filtration d'une composition fluide complexe selon l'une quelconque des revendications précédentes, **caractérisé en ce que** le piquage (36-1) traverse de façon étanche le fond (18) de l'embase (12), traverse de façon étanche la membrane (16) et comprend des perforations dans sa partie située au sein de la chambre haute C2 de façon à laisser passer le perméat.

## Patentansprüche

1. Vorrichtung zur Filtrierung einer komplexen Fluidzusammensetzung, wie beispielsweise Blut, **dadurch gekennzeichnet, dass** diese ein Unterteil (12) mit einem Boden (18), ein Oberteil (14) mit einem Boden (30) und eine Membran (16), die zwischen dem Unterteil und dem Oberteil derart angeordnet ist, dass eine untere Kammer C1 und eine obere Kammer C2 gebildet sind, und einen in die untere Kammer C1 mündenden Anschluss (26) für den Einlass der komplexen Zusammensetzung, einen in dieselbe untere Kammer C1 mündenden Anschluss (28) für den Auslass und einen Anschluss (36, 36-1) für den Auslass der oberen Kammer C2 aufweist, wobei der Anschluss (36, 36-1) für den Auslass der oberen Kammer C2 mit einer Vakuumquelle PV verbunden ist.

2. Vorrichtung zur Filtrierung einer komplexen Fluidzusammensetzung nach Anspruch 1, **dadurch gekennzeichnet, dass** das Unterteil (12) Rippen (24) aufweist, die eine Anordnung von Schikanen bilden.

3. Vorrichtung zur Filtrierung einer komplexen Fluidzusammensetzung nach Anspruch 2, **dadurch gekennzeichnet, dass** die Rippen (24) im rechten Winkel zum Anschluss (26) für den Einlass ausgerichtet sind.

4. Vorrichtung zur Filtrierung einer komplexen Fluidzusammensetzung nach Anspruch 1, 2 oder 3, **dadurch gekennzeichnet, dass** das Oberteil (14) Rippen (34) aufweist, die eine Anordnung von Schikanen bilden.

5. Vorrichtung zur Filtrierung einer komplexen Fluidzusammensetzung nach den Ansprüchen 3 und 4, **dadurch gekennzeichnet, dass** die Rippen (24) des Unterteils (12) und die Rippen (34) des Oberteils (14) gegenüberliegend angeordnet sind, um die Membran (16) ruhigzustellen.

6. Vorrichtung zur Filtrierung einer komplexen Fluidzusammensetzung nach einem der vorhergehenden Ansprüche, **dadurch gekennzeichnet, dass** das Unterteil (12) einen Rand (22) aufweist, der dazu eingerichtet ist, die ringförmige Wand (32) des Oberteils (14) aufzunehmen, um die Ruhigstellung der Membran (16) entlang dem Umfang zu gewährleisten.

7. Vorrichtung zur Filtrierung einer komplexen Fluidzusammensetzung nach einem der vorhergehenden Ansprüche, **dadurch gekennzeichnet, dass** der Anschluss (36-1) auf dichte Weise den Boden (18) des Unterteils (12) und die Membran (16) durchquert und in seinem Abschnitt, die sich im Bereich der oberen Kammer C2 befindet, Perforierungen aufweist, um das Permeat hindurchzulassen.

## Claims

1. A device for filtering a complex fluid composition such as blood, **characterised in that** it comprises a base part (12) with a base (18), a head part (14) with a base (30), and a membrane (16) disposed between said base part and said head part so as to define a lower chamber C1 and an upper chamber C2, a tapping (26) for entry of the complex composition emerging in the lower chamber C1, an outlet tapping (28) emerging from this same lower chamber C1 and an outlet tapping (36, 36-1) from the upper chamber C2, the outlet tapping (36, 36-1) from the upper chamber C2 being connected to a vacuum source PV.

2. A device for filtering a complex fluid composition according to claim 1, **characterised in that** the base part (12) comprises ribs (24) forming a set of baffles.

3. A device for filtering a complex fluid composition according to claim 2, **characterised in that** the ribs (24) are oriented perpendicular to the inlet tapping (26).

4. A device for filtering a complex fluid composition according to claim 1, 2 or 3, **characterised in that** the head part (14) comprises ribs (34) forming a set of baffles.

5. A device for filtering a complex fluid composition according to claims 3 and 4, **characterised in that** the ribs (24) on the base part (12) and the ribs (34) on the head part (14) are facing each other so as to immobilise the membrane (16).

6. A device for filtering a complex fluid composition according to any one of the preceding claims, **characterised in that** the base part (12) comprises a rim (22) intended to receive the annular wall (32) of the head part (14) so as to ensure the peripheral immobilisation of the membrane (16).

7. A device for filtering a complex fluid composition according to any one of the preceding claims, **characterised in that** the tapping (36-1) passes sealingly through the base (18) of the base part (12), passes sealingly through the membrane (16) and comprises perforations in the part thereof situated inside the upper chamber C2 so as to allow the permeate to pass.
